# EUROPÄISCHE PATENTSCHRIFT

(11) **EP 3 054 908 B1**
(45) Veröffentlichungstag und Bekanntmachung des Hinweises auf die Patenterteilung: **04.04.2018**
(21) Anmeldenummer: 14781596.3
(22) Anmeldetag: 09.10.2014
(51) Int. Cl.: A61F 13/02

(54) **TRÄGERSYSTEM FÜR EIN KÖRPERGETRAGENES OBJEKT UND HERSTELLUNGSVERFAHREN**
CARRIER SYSTEM FOR A BODY MOUNTED OBJECT AND METHOD FOR MANUFACTURING SAME
SYSTÈME DE SUPPORT POUR UN OBJET PORTÉ PAR UN CORPS ET PROCÉDÉ DE FABRICATION

(30) Priorität: 10.10.2013 EP 13188144
(43) Veröffentlichungstag der Anmeldung: 17.08.2016
(73) Patentinhaber: Roche Diabetes Care GmbH, 68305 Mannheim (DE); F. Hoffmann-La Roche AG, 4070 Basel (CH)
(72) Erfinder: GOLDBERG, Norman, 40591 Düsseldorf (DE); HERBERTZ, Michael, 42929 Wermelskirchen (DE); KUBE, Oliver, 67549 Worms (DE); NITTENWILM, Ralf, 56203 Höhr-Grenzhausen (DE); WALTER, Helmut, 64646 Heppenheim (DE)
(74) Vertreter: Pfiz, Thomas
(86) Internationale Anmeldenummer: PCT/EP2014/071671
(87) Internationale Veröffentlichungsnummer: WO 2015/052288

(56) Entgegenhaltungen:
- EP-A1- 2 636 417
- US-A1- 2009 076 453
- US-A1- 2009 216 103
- US-A1- 2011 275 997

## Beschreibung

Die Erfindung betrifft ein Trägersystem für ein körpergetragenes Objekt, insbesondere medizinisches Instrument mit einem flexiblen Trägerpflaster, das eine flächige Trägerlage und eine auf deren Unterseite aufgebrachte, durch Andrücken auf der Haut eines Körperteils haftende Klebstoffschicht aus einem Haftklebstoff aufweist, und mit einer auf der hautabgewandten Oberseite der Trägerlage angeordneten, insbesondere starren Montageplattform, wobei die Oberseite der Trägerlage mit einer zugewandten unteren Fügefläche der Montageplattform flächig verbunden ist. Die Erfindung betrifft weiter ein Verfahren zur Herstellung sowie die besondere Verwendung eines derartigen Trägersystems.

Allgemein verlangen solche medizinische Vorrichtungen und Anwendungen einen höchstmöglichen Grad an Qualität und Verlässlichkeit. Die hierbei eingesetzten Materialien müssen vielfältigen Erfordernissen genügen: Sie müssen beispielsweise biologisch kompatibel bzw. inert sein sowie korrosions- und temperaturresistent sein und sie müssen unter den unterschiedlichsten Bedingungen fehlerfrei funktionieren. Daraus ergeben sich strenge Anforderungen an solche Systeme und deren Herstellung. Beim Einsatz von Klebstoffen wie auch allen anderen im medizinischen Bereich direkt am menschlichen Körper eingesetzten Materialien sind nicht nur höchste Qualitätsanforderungen, sondern auch vielfältige gesetzliche Vorschriften zu berücksichtigen; gleiches gilt für den gesamten Herstellungsprozess.

Im Bereich der Medizintechnik im Umfeld von kontinuierlichem Glukose-Monitoring (CGM), Insulinabgabesystemen (IDS) und Elektroenzephalografie(EEG)-Sonden ist eine Verbindung von Trägerpflaster und Instrument mittels Ultraschallschweißen bekannt. Hierbei kommen "Standard-Pflaster" mit Vlies zum Einsatz, bei denen eine Tragedauer von bis zu 4 Tagen und in manchen Fällen auch 5 Tagen erreicht wird. Solche Standard-Pflaster besitzen einen maximalen Haftkleberauftrag von weniger als 80g/m². Durch die lange Tragedauer oder hohe Last am Körper lösen sich die Pflaster sukzessive vom Rand her ab. Die Probanden müssen speziell bei langer Tragdauer immer wieder die Pflaster nachkleben, überkleben oder aber die Pflaster versagen vorzeitig. Ursache dieses frühen Versagens ist sowohl die Verwendung von Standard-Pflastern, die für eine solch lange Tragezeit nicht konzipiert sind, als auch die Verbindungstechnik "Ultraschallschweißen". Hierbei wird punktuell eine hohe Wärme eingebracht, die dazu führt, dass das aus Kunststoff bestehende Trägermaterial leicht aufschmilzt (T größer als l30°C). Dies führt aber zu einer lokalen Zersetzung des Trägermaterials, wodurch die wirksame Klebefläche reduziert wird und sich im Klebefilm innere Spannungen aufbauen. Da es sich um visko-elastische Systeme handelt, führt dies zur Ablösung des Pflasters vom Körper. Als Resultat ist die wirksame Klebefläche des Pflasters zum Körper verringert. Dies ist häufig im Bereich unter dem medizinischen Instrument der Fall, welcher für die vorgesehene Funktion, beispielsweise Kontakthaftung einer Hautelektrode, eigentlich besonders gut auf der Haut haften sollte.

Aus der EP-A 1 923 081 ist eine Infusionsvorrichtung für Insulin bekannt, die einen Kathederkopf aufweist, der über ein Pflaster auf der Haut fixierbar ist und an seiner Oberseite einen Konnektor zur lösbaren und rotierbaren Steckverbindung einer Insulinpumpe besitzt, wobei die vollständige Körperbefestigung erst durch direkte Klebebefestigung von über das Pflaster überstehenden peripheren Bereichen der Insulinpumpe auf der Haut erfolgt. Dabei sollen nicht näher spezifizierte hautverträgliche Klebemittel zu Einsatz kommen. Damit lässt sich eine tragegerechte Ausrichtung der Pumpe am Körper bewerkstelligen, wobei jedoch für die sichere Befestigung der Einsatz und die Handhabung zusätzlicher Hautklebemittel am Anwendungsort erforderlich sind.

Die US 2011/257997 A1 beschreibt eine Vorrichtung zur Medikation eines Patienten mit einem zweiteiligen Gehäuse aus einem Basisteil zum Anbringen auf der Haut und einem damit verbindbaren beweglichen Gehäuseoberteil, wobei die beiden Teile über einen mechanischen Formschluss lösbar verbunden oder ggf. verklebt sind. Als Fügebereich sind nur abstehende Formteilstrukturen offenbart, welche die Herstellung und Montage eher aufwändig gestalten.

Die US 2009/0216103 A1 offenbart ein flexibles Trägerpflaster, dessen Oberseite mit einer zugewandten Fügefläche einer Montageeinheit flächig verbunden ist, und dessen Unterseite eine auf der Haut haftende Klebstoffschicht aufweist. Dieses Dokument befasst sich mit der Ausbildung der Trägerlage, macht aber keine näheren Angaben zu der Klebstoffschicht.

Ausgehend hiervon liegt der Erfindung die Aufgabe zugrunde, die im Stand der Technik bekannten Systeme und Verfahren weiter zu verbessern und eine hohe Standfestigkeit für eine möglichst langdauernde und zuverlässige Tragefunktion bei gleichzeitig vereinfachter Herstellbarkeit und unkomplizierter Applikationsmöglichkeit anzugeben.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine Verbindungsart für das zu tragende Objekt vorzusehen, welche die Haftklebeschicht des Pflasters möglichst wenig beeinträchtigt. Dementsprechend wird erfindungsgemäß vorgeschlagen, dass ein Füge- bzw. Strukturbereich der Montageplattform über eine strukturelle (konstruktive) Klebverbindung aus einem Strukturklebstoff fest mit der Oberseite der Trägerlage verbunden ist, und dass die Klebstoffschicht einen Haftklebstoffauftrag von 80 bis 120 g/m² aufweist. Ein solcher fester Verbund durch strukturelles Kleben lässt sich nur zerstörend lösen, wohingegen Haftkleben an sich reversibel ist.

Als Strukturklebstoffe werden allgemein solche Klebstoffe bezeichnet, die in einer Fügeverbindung eingesetzt werden, welche dadurch einen wesentlichen Anteil an der Sicherstellung der Stabilität und/oder Funktion des Bauteils erlangt. Man spricht in diesem Zusammenhang auch von konstruktiven Verklebungen bzw. strukturellen Klebstoffen. Als Faustregel gilt, dass eine strukturelle Klebverbindung eine Scherfestigkeit von mehr als 2 N/mm² besitzt. Demgegenüber wird als Haftklebstoff eine Klebstoffart bezeichnet, die in lösemittelfreiem Zustand insbesondere bei Raumtemperatur eine Adhäsion mit viskoelastischen Stoffverhalten bietet und bei direktem Kontakt auf einer Vielzahl von Oberflächen haftet, wobei durch Andrücken an die Oberfläche der zu verklebenden Fügeteile eine Benetzung herbeigeführt wird, die ausreichende Haftungskräfte ergibt.

Durch die erfindungsgemäße strukturelle Klebverbindung zwischen Montageplattform bzw. Bodenplatte und Pflaster wird die Haftklebeschicht des Pflasters (Verbindung zum Körper) nicht zerstört. Dies bedeutet, dass unter dem getragenen Objekt bei hoher wirksamer Klebefläche die volle Klebkraft zur Verfügung steht. Es ergeben sich geringere Scherspannungen zwischen Körper und Pflaster aufgrund der aufrechterhaltenen Flexibilität des Trägermaterials. Auch die Randablösung des Pflasters kann verringert werden und es kann eine längere Tragedauer ohne Verlust des Systems erreicht werden. Einschränkungen des Nutzers in alltäglichen Lebenssituationen werden verringert und es wird aufgrund des verbesserten Tragekomforts eine bessere Akzeptanz erreicht.

Vorteilhafterweise besitzt das Trägerpflaster einen Schutz in Form einer die Klebstoffschicht klebeseitig abdeckende Abziehfolie (Release Liner als Abdeckung), die vor dem Aufkleben vorzugsweise mittels einer überstehenden Lasche (Abziehhilfe bzw. Anfasslasche) abziehbar ist. Dabei kann die Abziehfolie auch mehrteilig ausgebildet sein.

Eine weitere vorteilhafte Ausführung sieht vor, dass die Trägerlage an ihrer Oberseite bzw. Oberfläche mit einer zugewandten unteren Fügefläche der Montageplattform mittels Strukturklebstoff flächig verbunden ist, so dass eine einfache Montage und eine zuverlässige unlösbare Fixierung der Plattform ohne Beeinträchtigung der Haftklebeschicht erreicht wird.

Bevorzugt besteht die Trägerlage aus Folienmaterial oder Textilmaterial oder einem Schaum oder einer Kombination davon, so dass eine flächige Anpassung an eine unregelmäßige Hautkontur möglich ist.

Um eine hohe Flexibilität bei guter Wasserdampfdurchlässigkeit zu erreichen, ist es günstig, wenn die Trägerlage aus einer thermoplastischen (ein- oder mehrschichtigen), bevorzugt mikroperforierten Folie besteht.

Für eine hinreichende Dehnbarkeit sollte die Trägerlage ein maximales Elastizitätsmodul von weniger als 10 N/mm², vorzugsweise weniger als 5 N/mm² besitzen.

Vorteilhafterweise weist die Klebstoffschicht einen Haftklebstoffauftrag von 100 bis 110 g/m2 auf. Hintergrund ist, dass die menschliche Haut eine hohe Oberflächenrauigkeit (z.B. durch tote Hautpartikel, Riefen etc.) hat und der Kleber in tiefere Hautschichten eindringen (fließen) kann. Eine Erhöhung der Klebermenge auf mehr als 80g/m2 ist somit hilfreich, die Haftung zur menschlichen Haut zu verbessern und langandauernder zu gestalten.

Weiterhin ist es auch von Vorteil, wenn die Klebstoffschicht durch einen Haftklebstoff auf der Basis von Acrylat, Silikon, Kautschuk, thermoplastischem Elastomer, Hydrogel, Polyurethan, Polysiloxan, Vinylacetat oder Mischungen aus diesen gebildet ist. Bevorzugt wird ein Acrylat-Vinylacetat-Copolymer als Haftklebstoff eingesetzt.

Im Hinblick auf die Forderungen nach einer längeren Tragedauer bei dauerhaft guter Haftung auf der Haut sowie einer schmerz- und rückstandsfreien Ablösung spielt die Atmungsaktivität und damit die Wasserdampfdurchlässigkeit (MVTR, "Moisture Vapor Transmission Rate") eine entscheidende Rolle. Die Wasserdampfdurchlässigkeit kann dabei gemäß DIN EN 13726-2 bei einer Temperatur von 37°C und einer Luftfeuchtigkeit von 18% bestimmt werden. Vorteilhafterweise besitzt die Trägerlage und/oder die Klebstoffschicht des Trägerpflasters einen MVTR-Wert von mehr als 400 g/m2 in 24 h.

Um eine hohe Festigkeit zu erzielen, ist es vorteilhaft, wenn die strukturelle Klebverbindung einen Strukturklebstoff auf Basis von Cyanoacrylat-, Epoxid- oder Polyurethan-Klebstoff umfasst.

Aufgrund der langen Tragedauer am Körper sollten der Haftklebstoff und der Strukturklebstoff eine für medizinische Anwendungen zugelassene biokompatible Zusammensetzung aufweisen.

Vorteilhafterweise ist die Montageplattform durch ein Kunststoff-Formteil bevorzugt aus Polycarbonat gebildet.

Gegenstand der Erfindung ist auch ein medizinisches Instrument mit einem vorstehend beschriebenen Trägersystem.

In verfahrensmäßiger Hinsicht wird die eingangs genannte Aufgabe durch ein Verfahren zur Herstellung eines Trägersystems für ein körpergetragenes Objekt mit folgenden Schritten gelöst:
- Bereitstellen eines Trägerpflasters vorzugsweise als Stanzteil bestehend aus einer Trägerlage, einer Klebstoffschicht und einer Abziehfolie, wobei die Klebstoffschicht (32) einen Haftklebstoffauftrag von 80 bis 120 g/m² aufweist,
- Auftragen eines Strukturklebstoffs als Kleberaupe auf einen Fügebereich einer Montageplattform,
- Kontaktieren des Fügebereichs mit der hautabgewandten Oberseite des Trägerpflasters unter Bildung einer strukturellen Klebverbindung.

Um eine konstruktive stoffschlüssige Verbindung zu erzielen, ist es vorteilhaft, wenn die Montageplattform beim Aushärten des Strukturklebstoffs gegen das Pflaster angepresst wird.

Eine zusätzliche Verbesserung lässt sich dadurch erreichen, dass der Strukturklebstoff nach dem Kontaktieren gegebenenfalls durch Bestrahlung beschleunigt getrocknet bzw. ausgehärtet wird, so dass er seine volle Fügefestigkeit erhält.

Ein weiterer Erfindungsaspekt betrifft ein erfindungsgemäßesTrägersystem zur Verwendung für mindestens eine Anwendung aus der folgenden Gruppe
- sensorische Überwachung bzw. Erfassung eines Körperparameters,
- Verabreichung von Wirkstoffen, insbesondere Insulin an oder durch die Haut,
- Bereitstellung eines Körperzugangs oder Körperausgangs,
- Tragen von Körperschmuck.

Im Folgenden wird die Erfindung anhand der in der Zeichnung schematisch dargestellten Ausführungsbeispiele näher erläutert. Es zeigen:
- Fig. 1: ein Trägersystem mit einem medizinischen Instrument zur haftklebenden Fixierung auf der Haut in einer perspektivischen Ansicht;
- Fig. 2: das Trägersystem mit einer Montageplattform in einer Fig. 1 entsprechenden Darstellung;
- Fig. 3: eine Schnitt des Trägersystems entlang der Linie 3-3 der Fig. 2 im Klebezustand auf der Haut;
- Fig. 4: eine Ausschnittsvergrößerung der Fig. 3.

Das in der Zeichnung dargestellte Trägersystem 10 umfasst zur haftklebenden Fixierung eines medizinischen Instruments 12 für diagnostische Langzeitanwendungen auf der Haut 14 eines Körperteils 16 ein Trägerpflaster 18 und eine auf dessen hautabgewandter Oberseite angeordnete starre Montageplattform 20. Die selbstklebende Unterseite des Trägerpflasters 18 ist vor Gebrauch durch eine zweigeteilte Abziehfolie 22 abgedeckt, die mittels überstehender Abziehlaschen 24 einfach entfernbar ist.

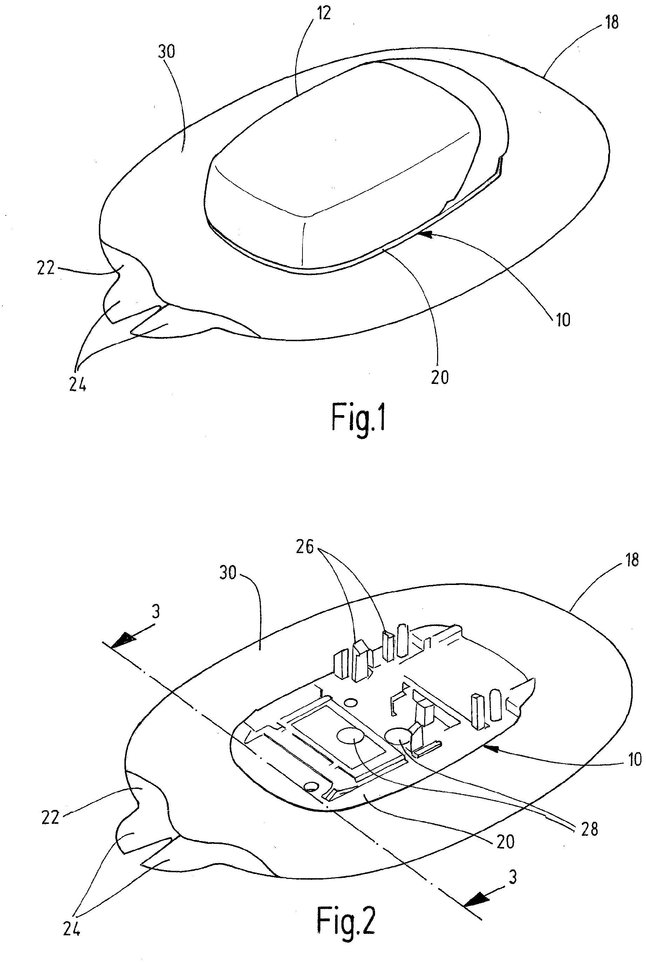

Wie aus Fig. 1 und 2 zu entnehmen, kann die Montageplattform 20 die Halterung des Instruments 12 über Formschlussmittel 26 ermöglichen. Die dauerhaft befestigte Montageplattform 20 kann auch integraler Bestandteil des Instruments 12 sein. Ein solches Instrument bzw. Gerät kann ein diagnostisches Messsystem beispielsweise zur kontinuierlichen Glukoseüberwachung, ein Sensor oder eine Abgabevorrichtung für Pharmazeutika beispielsweise in Form einer Insulinpumpe sein, oder auch eine elektronische Baugruppe wie einen RFID-Chip enthalten. Weitere denkbare Anwendungen bestehen in der Organüberwachung, z.B. der glomerulären Filtrationsrate zur Überprüfung der Nierenfunktion oder in der Durchführung von Laktattests.

In der gezeigten Ausführung ist die Montageplattform durch ein plattenartiges Kunststoff-Formteil beispielsweise aus Polycarbonat gebildet. Durchbrüche 28 in der Montageplattform 20 und dem darunterliegenden Pflasterbereich ermöglichen einen direkten Hautzugang bzw. Hautausgang.

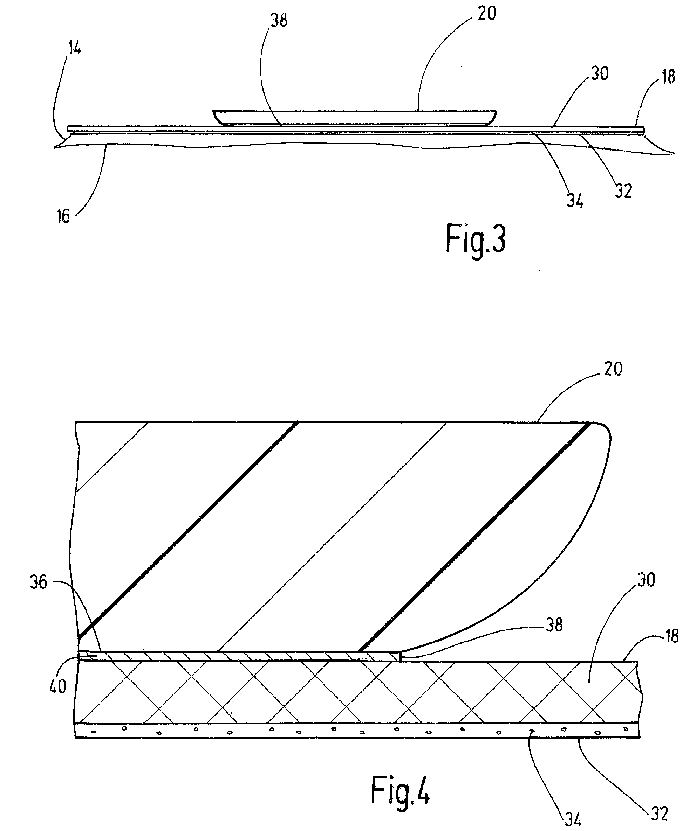

Wie am besten aus Fig. 3 und 4 ersichtlich, umfasst das Trägerpflaster 18 eine flächige flexible Trägerlage 30 und eine auf deren Unterseite aufgebrachte, durch Andrücken auf der Haut 14 haftende Klebstoffschicht 32 aus einem Haftklebstoff 34. Auf der hautabgewandten Oberseite der Trägerlage 30 ist die starre Montageplattform 20 in einem als untere Fügefläche ausgebildeten Strukturbereich 36 über eine konstruktive bzw. strukturelle Klebverbindung 38 aus einem Strukturklebstoff 40 dauerhaft fixiert.

Eine wesentliche Anforderung an ein auf die Haut klebbares Trägerpflaster 18 ist die Hautverträglichkeit bzw. Biokompatibilität des dafür verwendeten Klebstoffs beispielsweise gemäß DIN ISO 10993. Dazu zählt insbesondere auch ein möglichst geringer Restmonomergehalt, um nicht zytotoxisch oder hautirritierend zu wirken. Im Hinblick auf Langzeitanwendungen, beispielsweise Anwendungen über einen Zeitraum von bis zu sieben Tagen, kommen weitere Anforderungen hinzu, insbesondere die vom Prinzip her zunächst gegensätzlichen Forderungen nach einer raschen, dauerhaften, festen, sich auch im Alltag z.B. beim Sport, Dusch- oder Badevorgang oder auch in einer Sauna nicht ablösenden oder verrutschenden oder den Träger in irgendeiner Form behindernden Verklebung - die dazuhin auch noch druckfrei und die Mikrozirkulation der Haut nicht störend sein soll - und der Forderung nach einer guten, rückstandslosen Ablösbarkeit des Pflasters nach längerem Gebrauch.

Haftklebstoffe auf Acrylatbasis stellen eine in diesem Zusammenhang verwendbare Klasse von Klebstoffen dar. Der Vorteil dieser Materialien liegt in ihrer hervorragenden Haftung, der guten Verträglichkeit der Inhaltsstoffe zur menschlichen Haut, der geringen Neigung zur Sensibilisierung und der sehr guten Sterilisations- und Alterungsbeständigkeit.

Alternativ können hochmolekulare Acrylathaftklebstoffe in Verbindung mit weichmachenden Additiven als Klebstoffe eingesetzt werden. Der Einsatz der weichmachenden Inhaltsstoffe bewirkt in Zusammenhang mit der hochmolekularen Polymermatrix eine sichere Haftung auf der menschlichen Haut. Zu beachten ist hier jedoch die Migrationsneigung der weichmachenden Komponenten. Dies kann als Folge der Mischung zweier Komponenten mit unterschiedlichem Haftverhalten ein uneinheitliches Abziehverhalten von der Haut bedingen.

Für Verklebungen auf der Haut können auch Silikon-Gel-Klebstoffe eingesetzt werden. Diese haben den Vorteil, dass sie auch nach langer Tragezeit leicht und ohne Hautschädigung von der Haut abgezogen werden können. Gegenüber acrylatbasierenden Haftklebstoffen ist allerdings die Klebkraft und die Wasserdampfdurchlässigkeit reduziert. Auch ist die Herstellung von unterbrochenen Klebstoffschichten mit diesen Haftklebstoffen technologisch aufwändiger.

Eine weitere Klebstoffalternative für Verklebungen im medizinischen, dermatologischen oder kosmetischen Bereich stellen thermoplastische Elastomere auf der Basis eines Blockcopolymers mit einem Styrol-Olefin-Styrol-Block, einer klebrig machenden Komponente in Form eines Tackifiers sowie in der Regel einem Weichmacher und einem amphiphilen Copolymer dar. Hier ist zu berücksichtigen, dass Weichmachermigration sowohl die Klebkraft als auch das Erscheinungsbild eines Pflasters beeinträchtigen kann, und dass solche Klebstoffe u.U. einen okklusiven, d.h. die Haut hermetisch abschirmenden Effekt haben können.

Grundsätzlich einsetzbar für die vorliegenden Zwecke sind Klebstoffe auf Basis Synthesekautschuk, insbesondere auf Basis Polyisobutylen und dann bevorzugt als Mischung mit einem Styrol-Isopren-Styrol Blockcopolymer bzw. in Form eines Styrol-lsobutylenStyrol- Blockcopolymers. Derartigen Klebstoffen können zur Verbesserung ihrer klebenden Eigenschaften Additive wie insbesondere Harze zugesetzt werden.

Eine weitere Klasse von Klebstoffen für medikale Anwendungen sind Hydrogele. Sie enthalten einen hohen Anteil an Wasser und natürliche Polymere mit hohen Molekulargewichten, wie z.B. Polysaccharide wie Glucomannan, Galactomannan, Carrageenan oder Alginat oder auch synthetische, oft aus Kohäsionszwecken vernetzte Polymere, beispielsweise Copolymere auf Basis 2-acrylamid-2-methylpropan-Sulfonsäure.

Andere im vorliegenden Zusammenhang grundsätzlich einsetzbare Klebstoffe sind Klebstoffe auf Basis Polyurethan oder Polysiloxan.

Als die im Hinblick auf die genannten Anforderungen und insbesondere auch im Hinblick auf einen möglichst geringen Restmonomergehalt beste Klebstoffvariante hat sich ein Haftklebstoff umfassend ein oder mehrere Polymere, hergestellt aus olefinischen Monomeren, mit einem Restgehalt an freien Monomeren von weniger als 0,01 Gew.-% erwiesen. Die Polymere können dabei vorliegen als Homopolymer, Copolymer oder Blockpolymer oder als eine Mischung von verschiedenen Homo-, Co- und/oder Blockpolymeren. Sie können weitere Substanzen enthalten wie insbesondere Klebharze, bevorzugt hydriertes Kolophonium. Als besonders vorteilhaft im Hinblick auf die gewünschten Eigenschaften des Haftklebstoffs hat sich ein Copolymer aus 2-Ethylhexylacrylat und Vinylacetat herausgestellt. Da Ester der Acrylsäure α,β-ungesättigte Carbonylverbindungen sind, unterliegen sie der Michael-Addition. Für eine längere Tragedauer von sieben oder mehr Tagen sollte der Klebstoff nur eine geringe Restmonomer-Konzentration enthalten, um nicht zytotoxisch bzw. hautirritierend zu sein, was bei dem oben genannten Restmonomergehalt gegeben ist.

Im Hinblick auf die Forderungen nach einer längeren Tragedauer bei dauerhaft guter Haftung auf der Haut sowie einer schmerz- und rückstandsfreien Ablösung spielt auch die Atmungsaktivität und damit die Wasserdampfdurchlässigkeit (MVTR, "Moisture Vapor Transmission Rate") klebender Systeme eine entscheidende Rolle. Die Wasserdampfdurchlässigkeit wird dabei entweder in Anlehnung an DIN EN 13726-2 bei einer Temperatur von 37°C und einer Luftfeuchtigkeit von 18% bestimmt oder in Anlehnung an ASTM E 96 nach der "upright-cup"-Methode bei einer Temperatur von 23°C und einer Luftfeuchtigkeit von 50%.

Unter Wasserdampfdurchlässigkeit ist danach die Menge an Feuchtigkeit zu verstehen, die pro Zeiteinheit durch eine Membran als Wasserdampf hindurchgeleitet werden kann. Je höher der MVTR-Wert ist, desto effektiver kann Feuchtigkeit unter einem Wundverband verhindert werden. Da die Haut transpiriert und damit eine Feuchtigkeitsquelle ist, kann ein Mangel an Atmungsaktivität zu einer Überhydratisierung und als Folge davon zu einer Aufweichung der Haut führen oder auch einen nicht gewünschten Pflasterablösungsprozess fördern. Bei den herkömmlichen Pflastersystemen bestehend aus einer vor Gebrauch durch einen Liner abgedeckten Klebstoffschicht sowie einem Trägermaterial wurden bisher vorwiegend die Klebstoffkomponenten, die Dicke der Klebstoffschicht oder auch deren Struktur z.B. als Folge eines diskontinuierlichen Klebstoffauftrags als primär entscheidend im Hinblick auf die Wasserdampfdurchlässigkeit des gesamten Systems gesehen - der Träger selbst spielte dabei eine untergeordnete Rolle. Da jedoch die erfindungsgemäßen Trägerpflaster auf beiden Seiten abgedeckt sind, kommt der Beschaffenheit der Trägerlage 30 auch im Hinblick auf die MVTR-Werte des gesamten Systems eine deutlich größere Bedeutung zu.

Um mehrere Tage, insbesondere mindestens sieben Tage dauerhaft und unter allen möglichen Bedingungen verlässlich auf der Haut zu haften und dennoch gleichzeitig eine MVTR-Rate aufzuweisen, die ein Atmen der Haut ermöglicht und bei Feuchtigkeitseintrag nicht zum Ablösen des Pflasters führt, sollte der Haftklebstoffauftrag mindestens 80 g/m² betragen. Die obere Grenze liegt bei etwa 120 g/m², was ökonomische und verfahrenstechnische Gründe hat. Bevorzugt wird ein Haftklebstoffauftrag von 110 g/m2. Bei der daraus resultierenden Dicke der Klebstoffschicht liegt deren MVTR-Rate oberhalb ca. 400 g/m² in 24 h.

Die an den Haftklebstoff zur Verklebung auf der Haut gestellten Anforderungen treffen im Prinzip auch auf das zu verwendende Material der Trägerlage 30 zu: Auch wenn in diesem Zusammenhang eingesetzte Trägermaterialien keinen direkten Hautkontakt haben, so können doch Bestandteile von Trägermaterialien in die Klebstoffschicht migrieren bzw. sogar durch diese hindurchwandern und auf diese Weise in Kontakt mit der Haut kommen. Aus diesem Grunde sollten auch die Trägermaterialien nur Inhaltsstoffe enthalten, die hautverträglich und nicht oder wenig sensibilisierend sind. Weitere Anforderungen bestehen in den an sich gegensätzlichen Anforderungen einer gewissen Flexibilität bzw. Elastizität einerseits und einer gewissen Steifigkeit andererseits. Die Flexibilität und Elastizität ist notwendig für eine Anpassung an eine unregelmäßige Hautkontur und einen sicheren Stoffschluss auf der Haut auch bei Hautbewegungen, um dadurch der Haut keinen Widerstand entgegenzusetzen und ein Fremdkörpergefühl zu vermeiden. Die Steifigkeit ist notwendig im Hinblick auf den Stanzvorgang bei der Herstellung und auch zur Gewährleistung einer sicheren und raschen Befestigung des zu tragenden Objekts.

Trägermaterialien, die die genannten Anforderungen im Prinzip erfüllen, können z.B. aus einer Vielzahl von polymeren Zusammensetzungen bestehen, die in der Lage sind, reibungsarme, flexible Polymerfolien zu bilden, beispielsweise aus Polyolefinen wie Polyethylen, Polypropylen oder Polybutylen, aus Vinylcopolymeren wie Polyvinylchlorid oder Polyvinylacetat, aus Olefin-Copolymeren oder auch aus Acrylpolymeren und -copolymeren. Gemische oder Mischungen aus plastischen oder aus plastischen und elastomeren Materialien wie z.B. Polypropylen und Polyethylen, Polyurethan und wahlweise Polyolefin, Polycarbonat oder Polyester können gleichfalls Verwendung finden, ebenso reine TPE- oder TPU-Folien. Die Trägerlage 30 kann als ein- oder mehrschichtige Folie vorliegen. Sie kann mit an sich bekannten Verfahren zur Folienproduktion hergestellt werden, z.B. durch Extrusion, Koextrusion, Gießen aus Lösungsmittel, Aufschäumen.

Textile Materialien wie Gewirke, Gestricke oder Gewebe sowie auch Schäume kommen grundsätzlich ebenfalls als Material für die Trägerlage 30 in Frage, ebenso Kombinationen aus den genannten Materialien.

Im Hinblick auf die an sich gegensätzlichen Kriterien der Flexibilität und der Steifigkeit kann die Dicke der Trägerlage 30 im Bereich von 2µm bis 250µm liegen. Vorzugsweise weist die Trägerlage eine Dicke von weniger als 30µm auf. Die Wasserdampfdurchlässigkeit des Trägermaterials sollte mindestens 400 g/m2 in 24 h betragen.

Eine Folie als Trägerlage kann monolithisch sein, sofern sie die erforderliche Wasserdampfdurchlässigkeit aufweist. Die Trägerlage kann jedoch auch mikroporös oder in sonstiger Weise löchrig sein, um eine ansonsten wasserdampfundurchlässige Polymerfolie wasserdampfdurchlässig zu machen. Folien aus den genannten Materialien weisen grundsätzlich einen nur sehr geringen MVTR-Wert auf. Um dies zu beheben, können solche Folien perforiert werden und damit ein hinreichendes Maß an Wasserdampfdurchlässigkeit erhalten. Eine regelmäßige und gleichförmig mikroperforierte thermoplastische Folie aber limitiert die MVTR des hautseitigen Haftklebstoffes auf die infolge der Perforation offenen Flächen der Folie; daher sollte es das Ziel einer geeigneten Perforation sein, eine möglichst große wasserdampfdurchlässige Fläche bereitzustellen. Andererseits sollte die Perforation die im Hinblick auf die Fixierung des Instruments notwendige Steifigkeit der Folie nicht negativ beeinträchtigen und gleichzeitig sollten die Perforationsöffnungen auf der hautabgewandten Seite der Trägerfolie nicht oder in nur möglichst geringem Maße durch den Klebstoffauftrag für die strukturelle Klebverbindung beeinträchtigt werden.

Im Hinblick auf die gewünschte hohe MVTR-Rate kommt insbesondere eine mikroperforierte thermoplastische Folie mit wahlweise unterschiedlichen Perforationsgeometrien als Trägermaterial in Frage. Für den vorliegenden Zweck besonders geeignete Perforationsgeometrien sind gleichmäßig oder ungleichmäßig (z.B. mit einer Ausbuchtung) konisch gestaltete Geometrien, wobei die im Hinblick auf ihren Durchmesser größere Öffnung auf der hautzugewandten Seite liegen sollte, um dadurch eine möglichst große Fläche für die Durchlässigkeit von Wasserdampf aus der hautseitigen Klebstoffschicht zu gewährleisten und gleichzeitig das Eindringen von Klebstoff in die Öffnungen auf der hautabgewandten Seite möglichst zu verhindern. Diese größere Öffnung kann dreieckig, rund oder beliebig polygonal gestaltet sein. Die Perforationen haben bevorzugt die Form eines Kegelstumpfes. Es können auch mehrere unterschiedliche Perforationsgeometrien in einer Folie miteinander kombiniert werden.

Zur besseren Verankerung des Klebstoffs auf der Kunststofffolie kann diese vor Klebstoffauftrag einer entsprechenden Vorbehandlung in Form z.B. einer Corona- oder Plasma-Vorbehandlung oder dem Auftragen eines geeigneten Primers oder einer Ätzbehandlung unterzogen werden.

Eine weitere Alterative, um eine gute Verankerung des Klebstoffs auf dem Trägermaterial und einen hohen MVTR-Wert zu erreichen, besteht darin, ein mehrlagiges Trägermaterial einzusetzen, beispielsweise durch Kaschierung geeigneter Materialien mit einer mikroperforierten Folie oder durch Koextrusion und anschließende Mikroperforation unterschiedlicher Materialien in einer Folie. Im Falle einer Zusammenkaschierung einer Kunststofffolie mit einem anderen Material bieten sich Vliesstoffe oder andere Textilien aufgrund ihrer Wasserdampfdurchlässigkeit an. Die Kaschierung kann dabei mit einem der an sich bekannten Kaschierverfahren erfolgen, beispielsweise mittels Wärmeeintrag oder mittels eines Klebstoffs. Ebenfalls geeignet als Trägermaterial sind kunststoffbeschichtete Fasermaterialien, bei denen zumindest die Kunststoffbeschichtung entsprechend perforiert ist. Die textile Seite des Trägermaterials kann sich dabei je nach Eignung sowohl auf der hautzugewandten Seite des Trägermaterials als auch auf der hautabgewandten Seite befinden.

Neben den angesprochenen Kunststofffolien oder Kombinationen, die Kunststofffolien enthalten, sind auch reine Textilträger als Trägerlage 30 einsetzbar. Diese Textilien können in Form von Vliesstoffen oder in gewebter, genähter oder gestrickter Form eingesetzt werden; die Bindung der Fasern untereinander ist grundsätzlich beliebig. Das Ausgangsmaterial dieser Textilträger kann aus natürlichen oder synthetischen Polymerstoffen bestehen, beispielsweise Cellulose, Seide oder Baumwolle als natürliche Polymerstoffe oder Polyvinylchlorid, Polyurethan, Polyester, Polyamid, Nomex, Kevlar, Polypropylen, Polyacryl, Preox, Nylon oder Kunstseide als synthetische Polymerstoffe.

Als besonders vorteilhaft im Hinblick auf die hier vorliegenden Anforderungen an das Material der Trägerlage 30 hat sich ein querelastisches Polyestergewebe oder Polyestergewirke erwiesen. Diese Gewebe oder Gewirke erhalten ihre Elastizität durch die Elastizität der verwendeten Polyestergarne. Bei ebenfalls hier verwendbaren leicht elastischen Polyestervliesen ist zu bedenken, dass sie nur in sehr dünner Form ausreichend flexibel und elastisch sind, was ein Stanzen des Materials und die Befestigung einer Montageplattform darauf erschweren kann. Polyestergewebe bzw. Polyestergewirke aber haben selbst in sehr dicken Ausführungen (bis ca. 150 g/m²) eine für die Anwendung als Trägerschicht eines Pflasters ausreichende Dehnbarkeit und sind dann auch besser schneid- bzw. stanzbar. Die unidirektionale Querelastizität des Polyestergewebes wird gemessen in Anlehnung an DIN 61632. Die Elastizität des eingesetzten Materials liegt zweckmäßig unter 150%, bevorzugt im Bereich von 20 bis 80%, besonders bevorzugt zwischen 40 und 70% und idealerweise zwischen 44 und 56%. Die Porosität des Trägermaterials, d.h. der Flächenanteil von Poren mit einer Fläche von weniger als ca. 400 µm² an der jeweiligen Bezugsfläche, liegt im Bereich von 10 bis 50%. Diese relative Porenfläche kann durch Messung und Zählen der Poren einer beliebigen ungedehnten Bezugsfläche ermittelt werden.

Im Falle eines Gewebes sollte die Rückschicht eine Kettfadenzahl von 300 bis 350 aufweisen, bevorzugt von 310 bis 330 und/oder eine Schussfadenzahl von 100 bis 140, bevorzugt 120 bis 130, jeweils gemessen je 10 cm ungedehntes Gewebe.

Eine weitere Komponente des erfindungsgemäßen Trägersystems bildet schließlich die auf der hautabgewandten Seite des Trägermaterials strukturell angeklebte Montageplattform 20, die als Basisplatte des diagnostischen Messinstruments 12 fungiert. Das Instrument 12 kann z.B. eine Kanüle führen, die als Halbimplantat sicher im bzw. am Körper positioniert werden muss. Der Verbund aus Trägerlage 30 und Montageplattform 20 ist während der Anwendung über die täglichen Körperbewegungen für einen längeren Zeitraum starken mechanischen Belastungen ausgesetzt und muss daher strukturell sichergestellt sein.

Für diesen Zweck kommen Strukturklebstoffe in Form von Epoxidharzklebstoffe, Cyanacrylate und Polyurethanklebstoffe in Frage. Bevorzugt werden Cyanacrylatklebstoffe: Sie sind für den Einsatz in der Medizintechnik geeignet und zugelassen, sie weisen eine sehr gute Haftung auf unterschiedlichen Materialien auf, auch auf Oberflächen mit geringer Oberflächenspannung, sie sind insbesondere geeignet für Kunststoff-Kunststoff-Verbindungen und für Kunststoff-Metall- Verbindungen, sie sind einkomponentig und daher leicht handhabbar, sie sind lösemittelfrei und sie härten sehr schnell aus. Einflussfaktoren bei der Aushärtung von Cyanacrylatklebstoffen sind insbesondere die relative Luftfeuchtigkeit bzw. Umgebungstemperaturen, der ph-Wert der Klebeflächen, die zu verbindenden Werkstoffe, die Breite des Klebespalts, der Aktivator, die Viskosität und die Reinheit der Oberfläche. Solche Klebstoffe werden insbesondere bei Anwendungen eingesetzt, bei denen eine gleichmäßige Spannungsverteilung und hohe Zug- sowie Scherfestigkeit gefordert werden.

Als besonders vorteilhaft erwiesen sich wegen ihrer Aushärtungsgeschwindigkeit und Oberflächentrocknung UV- und feuchtigkeitsvernetzende Cyanacrylate. So zeigte sich ein Cyanacrylat auf Basis "Ethyl-Cyanacrylat" besonders geeignet zur strukturellen Verklebung von Kunststoffen, Metallen und Elastomeren. Unter normalen Bedingungen wird der Aushärtungsprozess durch Luftfeuchtigkeit ausgelöst. Die volle Funktionsfähigkeit wird innerhalb relativ kurzer Zeit erreicht, der Aushärtvorgang bis hin zur vollen Medienbeständigkeit dauert jedoch noch mindestens 24h. So wird die Haftfestigkeit des Klebstoffs (Scherfestigkeit von 0,1 N/mm²) bei einer Temperatur von 22°C und 50% rel. Luftfeuchtigkeit in Abhängigkeit vom Material in einer Zeit von 3 bis maximal 300 Sekunden erreicht. Die Aushärtgeschwindigkeit ist abhängig von der relativen Luftfeuchtigkeit. Niedrigere Luftfeuchtigkeit verlangsamt die Aushärtung, durch eine höhere wird sie beschleunigt, die Endfestigkeit der Klebung kann dadurch jedoch nicht beeinträchtigt werden.

Ist die Aushärtgeschwindigkeit infolge großer Fügespalte relativ langsam, so kann durch den Einsatz eines Aktivators die Aushärtung beschleunigt werden. Dadurch kann sich allerdings die Endfestigkeit der Klebung verringern.

Die erfindungsgemäßen Trägersysteme zur Verklebung auf der Haut weisen darüber hinaus eine Abziehfolie 22 ("Release Liner") auf, welche die hautseitige Klebstoffschicht 34 vor Gebrauch abdeckt. Die Klebfläche kann vollständig von einer einteiligen Abziehfolie abgedeckt werden, es können aber auch mehrere Folienstreifen die Klebfläche jeweils nur partiell abdecken, wobei bevorzugt ist, dass die Klebfläche in jedem Fall vollständig abgedeckt ist. Geeignete Materialien für solche Abziehfolien sind silikonisiertes Papier oder gegebenenfalls klebstoffabweisend beschichtete Kunststofffolien aus beispielsweise Polyester, Polyethylen, Polyethylenterephthalat. Im Falle einer silikonisierten Abziehfolie sollte die Silikonisierung auch den einschlägigen lebensmittel- und medizinrechtlichen Bestimmungen entsprechen.

Die Herstellung des Trägersystems vollzieht sich in folgenden Schritten:
Zunächst wird das Trägerpflaster 18 als Schnitt- bzw. Stanzteil hergestellt. Dazu wird die Breitware des Grundmaterials, das in der Form eines Hautpflasters vorliegt, in Schmalrollen längs geschnitten. Diese Rollen werden in einem Rotationsstanzautomaten mit zwei Rotationswerkzeugen kontinuierlich verarbeitet. Ein 50 µm dicker Polyesterliner wird mit einem schwach haftenden Prozesstape zusammen kaschiert und im ersten Werkzeug geschlitzt, so dass sich eine Anfasslasche 24 ergibt. Das Pflaster wird dann inline auf den Polyesterliner zukaschiert, wobei die Originalabdeckung entfernt wird. Im zweiten Werkzeug wird die Außenkontur des Pflasters angestanzt und entgittert, außerdem werden Positionierungslöcher 28 durchgestanzt. Der Prozess wird mit einer Registersteuerung synchronisiert. Das Produkt liegt nun mit der Abdeckung mit einem definierten Rapport auf dem Prozesstape und kann weiter verarbeitet werden.

In einem weiteren Verfahrensschritt erfolgt die Verbindung des Stanzteils mit der Montageplattform 20. Dazu erfolgt zunächst auf der Montageplattform eine Vorbehandlung durch Auftrag eines Primers oder eines Hydrophilierungsmittels, bevor eine Kleberaupe des feuchtigkeitsabbindenden Cyanacrylatklebstoffs mit medizinischer Zulassung aufgetragen wird. Hierbei sind Cyanacrylate mit zusätzlicher UV-Vernetzung hinsichtlich der Reaktionszeit von Vorteil. Anschließend werden die VOC-Emissionen des Klebstoffs durch eine Absauganlage abgesaugt.

In einem weiteren Schritt wird das Pflaster vom Liner mittels eines Spendegeräts in eine Zwischenablage gespendet und dort positioniert. Ein Wende-Umsetzer entnimmt nun das Pflaster und wendet es um 180 Grad. Ein weiterer Umsetzer entnimmt das gewendete Pflaster und setzt es auf die mit der Klebstoffraupe benetzte Montageplattform ab und presst das Pflaster auf das Bauteil. Optional wird die nun erfolgende Härtung rückseitig mit einem UV-LED-Flächenstrahler-System beschleunigt.

Das derart hergestellte Trägersystem wird schließlich in einen Bauteilträger abgesetzt und anschließend je nach Klebstofftyp bis zu 24 h in einer Ablage bei konstantem Klima (Temperatur, Luftfeuchtigkeit) durchgehärtet.

Verwendung können erfindungsgemäße Systeme finden beispielsweise zur Überwachung aller möglichen Arten von Körperfunktionen wie Blutzucker, GFR, Blutdruck, EEG, Puls bzw. allgemein allen Arten von klinischen Parametern, als Körpereingangssysteme zur Verabreichung von Medikamenten oder Nahrung über Infusionen oder Pumpensysteme, als Körperausgangssysteme für Urinbeutel- oder Stomabefestigungen oder auch als Trägersystem für Körperschmuck.

## Patentansprüche

1. Trägersystem für ein körpergetragenes Objekt (12), insbesondere medizinisches Instrument mit einem flexiblen Trägerpflaster (18), das eine flächige Trägerlage (30) und eine auf deren Unterseite aufgebrachte, durch Andrücken auf der Haut (14) eines Körperteils (16) haftende Klebstoffschicht (32) aus einem Haftklebstoff (34) aufweist, und mit einer auf der hautabgewandten Oberseite der Trägerlage (30) angeordneten Montageplattform (20), wobei die Oberseite der Trägerlage (30) mit einer zugewandten unteren Fügefläche der Montageplattform (20) flächig verbunden ist, **dadurch gekennzeichnet, dass** ein Fügebereich (36) der Montageplattform (20) über eine strukturelle Klebverbindung (38) aus einem Strukturklebstoff (40) fest mit der Oberseite der Trägerlage (30) verbunden ist, und dass die Klebstoffschicht (32) einen Haftklebstoffauftrag von 80 bis 120 g/m² aufweist.

2. Trägersystem nach Anspruch 1, **dadurch gekennzeichnet, dass** das Trägerpflaster (18) vor Aufkleben eine die Klebstoffschicht (32) klebeseitig abdeckende, vorzugsweise mittels einer überstehenden Lasche (24) abziehbare Abziehfolie (22) aufweist.

3. Trägersystem nach Anspruch 1 oder 2, **dadurch gekennzeichnet, dass** die Trägerlage (30) aus Folienmaterial oder Textilmaterial oder einem Schaum oder einer Kombination davon besteht.

4. Trägersystem nach einem der Ansprüche 1 bis 3, **dadurch gekennzeichnet, dass** die Trägerlage (30) aus einer bevorzugt mikroperforierten thermoplastischen Folie besteht.

5. Trägersystem nach einem der Ansprüche 1 bis 4, **dadurch gekennzeichnet, dass** die Trägerlage (30) ein maximales Elastizitätsmodul von weniger als 10 N/mm², vorzugsweise weniger als 5 N/mm² besitzt.

6. Trägersystem nach einem der Ansprüche 1 bis 5, **dadurch gekennzeichnet, dass** die Klebstoffschicht (32) einen Haftklebstoffauftrag von 100 bis 110 g/m² aufweist.

7. Trägersystem nach einem der Ansprüche 1 bis 6, **dadurch gekennzeichnet, dass** die Klebstoffschicht (32) durch einen Haftklebstoff (34) auf der Basis von Acrylat, Silikon, Kautschuk, thermoplastischem Elastomer, Hydrogel, Polyurethan, Polysiloxan, Vinylacetat oder Mischungen aus diesen gebildet ist.

8. Trägersystem nach einem der Ansprüche 1 bis 7, **dadurch gekennzeichnet, dass** die Trägerlage (30) und/oder die Klebstoffschicht (32) des Trägerpflasters (18) einen MVTR-Wert von mehr als 400 g/m² Wasserdampfdurchlässigkeit in 24 h aufweist.

9. Trägersystem nach einem der Ansprüche 1 bis 8, **dadurch gekennzeichnet, dass** die strukturelle Klebverbindung (38) einen Strukturklebstoff (40) auf Basis von Cyanoacrylat-, Epoxid- oder Polyurethan-Klebstoff umfasst.

10. Trägersystem nach einem der Ansprüche 1 bis 9, **dadurch gekennzeichnet, dass** der Haftklebstoff (34) und der Strukturklebstoff (40) eine für medizinische Anwendungen zugelassene biokompatible Zusammensetzung aufweisen.

11. Trägersystem nach einem der Ansprüche 1 bis 10, **dadurch gekennzeichnet, dass** die Montageplattform (20) durch ein Kunststoff-Formteil bevorzugt aus Polycarbonat gebildet ist.

12. Verfahren zur Herstellung eines Trägersystems für ein körpergetragenes Objekt (12) nach einem der vorhergehenden Ansprüche mit folgenden Schritten:
- Bereitstellen eines Trägerpflasters (18) vorzugsweise als Stanzteil bestehend aus einer Trägerlage (30), einer Klebstoffschicht (32) und gegebenenfalls einer Abziehfolie (22), wobei die Klebstoffschicht (32) einen Haftklebstoffauftrag von 80 bis 120 g/m² aufweist,
- Auftragen eines Strukturklebstoffs (40) als Kleberaupe auf einen Fügebereich (36) einer Montageplattform (20),
- Kontaktieren des Fügebereichs (36) mit der hautabgewandten Oberseite des Trägerpflasters (18) unter Bildung einer strukturellen Klebverbindung (38), wobei die Oberseite der Trägerlage (30) mit einer zugewandten unteren Fügefläche der Montageplattform (20) flächig verbunden wird.

13. Verfahren nach Anspruch 12, **dadurch gekennzeichnet, dass** das Trägerpflaster (18) beim Aushärten des Strukturklebstoffs (40) gegen die Montageplattform (20) angepresst wird.

14. Verfahren nach Anspruch 12 oder 13, **dadurch gekennzeichnet, dass** der Strukturklebstoff (40) vorzugsweise durch Bestrahlung beschleunigt ausgehärtet wird.

15. Trägersystem (10) nach einem der Ansprüche 1 bis 11 zur Verwendung für mindestens eine Anwendung aus der folgenden Gruppe
- sensorische Überwachung bzw. Erfassung eines Körperparameters,
- Verabreichung von Wirkstoffen, insbesondere Insulin an oder durch die Haut (14),
- Bereitstellung eines Körperzugangs oder Körperausgangs,
- Tragen von Körperschmuck.

## Claims

1. A carrier system for a body-worn object (12), in particular a medical instrument with a flexible carrier plaster (18) comprising a sheetlike carrier ply (30) and on the lower side thereof an adhesive layer (32) comprising a pressure sensitive adhesive (34) and adherent to the skin (14) of a body part (16) by contact pressure, and with an assembly platform (20) arranged on the skin remote upper side of the carrier ply (30), wherein the upper side of the carrier ply (30) is attached flatly to a facing lower joining surface of the assembly platform (20), **characterized in that** a joining region (36) of the assembly platform (20) is firmly connected to the upper side of the carrier ply (30) via a structural type of adhesive bond (38) comprising a structural adhesive (40), and that the adhesive layer (32) has a pressure sensitive adhesive application of 80 to 120 g/m².

2. The carrier system as claimed in claim 1, **characterized in that** before the carrier plaster (18) is attached a release liner (22), preferably one removable by means of a projecting tab (24), covers the adhesive layer (32) on its sticky side.

3. The carrier system as claimed in claim 1 or 2, **characterized in that** the carrier ply (30) consists of foil material or textile material or a foam or a combination thereof.

4. The carrier system as claimed in any of claims 1 to 3, **characterized in that** the carrier ply (30) consists of a preferably microperforated thermoplastic foil.

5. The carrier system as claimed in any of claims 1 to 4, **characterized in that** the carrier ply (30) has a maximum elastic modulus of less than 10 N/mm², preferably less than 5 N/mm².

6. The carrier system as claimed in any of claims 1 to 5, **characterized in that** the adhesive layer (32) has a pressure sensitive adhesive application of 100 to 110 g/m².

7. The carrier system as claimed in any of claims 1 to 6, **characterized in that** the adhesive layer (32) is formed by a pressure sensitive adhesive (34) based on acrylate, silicone, rubber, thermoplastic elastomer, hydrogel, polyurethane, polysiloxane, vinyl acetate or mixtures thereof.

8. The carrier system as claimed in any of claims 1 to 7, **characterized in that** the carrier ply (30) and/or the adhesive layer (32) of the carrier plaster (18) has an MVTR value of more than 400 g/m² of water vapor permeability in 24 h.

9. The carrier system as claimed in any of claims 1 to 8, **characterized in that** the structural type of adhesive bond (38) comprises a structural adhesive (40) based on cyanoacrylate adhesive, epoxy adhesive or polyurethane adhesive.

10. The carrier system as claimed in any of claims 1 to 9, **characterized in that** the pressure sensitive adhesive (34) and the structural adhesive (40) have a biocompatible composition approved for medical applications.

11. The carrier system as claimed in any of claims 1 to 10, **characterized in that** the assembly platform (20) is formed by a plastic molding preferably of polycarbonate.

12. A method of producing a carrier system for a body-worn object (12) as claimed in any preceding claim, comprising the steps of:
- providing a carrier plaster (18) preferably as a die-cut part consisting of a carrier ply (30), an adhesive layer (32) and optionally a release liner (22), wherein the adhesive layer (32) has a pressure sensitive adhesive application of 80 to 120 g/m²,
- applying a structural adhesive (40) as an adhesive bead to a joining region (36) of an assembly platform (20),
- contacting the joining region (36) with the skin-remote upper side of the carrier plaster (18) to form a structural type of adhesive bond (38), wherein the upper side of the carrier ply (30) attaches flatly to a facing lower joining area (36) of the assembly platform (20).

13. The method as claimed in claim 12, **characterized in that** the carrier plaster (18) is pressed against the assembly platform (20) during the cure of the structural adhesive (40).

14. The method as claimed in claim 12 or 13, **characterized in that** the structural adhesive (40) is cured in a forced manner preferably by irradiation.

15. Carrier system (10) as claimed in any of claims 1 to 11 for use in at least one application from the following group:
- sensorially monitoring and/or capturing a body parameter,
- administering active ingredients, in particular insulin to or through the skin (14),
- providing a body entry port or body exit port,
- wearing body jewelry.

## Revendications

1. Système de support pour un objet (12) porté par un corps, en particulier instrument médical avec un patch de support souple (18) qui comprend une couche de support plane (30) et une couche d'adhésif (32) à base d'un adhésif sensible à la pression (34), appliquée sur la face inférieure de ladite couche de support, et adhérant par pression exercée sur la peau (14) d'une partie corporelle (16), et avec une plate-forme de montage (20) disposée sur la face supérieure, opposée à la peau, de la couche de support (30), dans lequel la face supérieure de la couche de support (30) est reliée dans le même plan à une surface d'assemblage inférieure, lui faisant face, de la plate-forme de montage (20), **caractérisé en ce qu'**une zone d'assemblage (36) de la plate-forme de montage (20) est reliée solidement à la face supérieure de la couche de support (30) par l'intermédiaire d'une liaison adhésive structurale (38) à base d'un adhésif structural (40), et **en ce que** la couche d'adhésif (32) comprend une charge d'adhésif sensible à la pression allant de 80 à 120 g/m².

2. Système de support selon la revendication 1, **caractérisé en ce que** le patch de support (18) comprend avant le collage une feuille détachable (22) recouvrant la couche d'adhésif (32) du côté adhésif, pouvant être détachée de préférence au moyen d'une languette saillante (24).

3. Système de support selon la revendication 1 ou la revendication 2, **caractérisé en ce que** la couche de support (30) est constituée d'une matière de feuille ou d'une matière textile ou d'une mousse ou d'une combinaison de celles-ci.

4. Système de support selon l'une des revendications 1 à 3, **caractérisé en ce que** la couche de support (30) est constituée d'une feuille thermoplastique de préférence microperforée.

5. Système de support selon l'une des revendications 1 à 4, **caractérisé en ce que** la couche de support (30) possède un module d'élasticité maximal de moins de 10 N/mm², de préférence de moins de 5 N/mm².

6. Système de support selon l'une des revendications 1 à 5, **caractérisé en ce que** la couche d'adhésif (32) comprend une charge d'adhésif sensible à la pression allant de 100 à 110 g/m².

7. Système de support selon l'une des revendications 1 à 6, **caractérisé en ce que** la couche d'adhésif (32) est formée à partir d'un adhésif sensible à la pression (34) à base d'acrylate, de silicone, de caoutchouc, d'élastomère thermoplastique, d'hydrogel, de polyuréthane, de polysiloxane, d'acétate de vinyle ou de mélanges de ceux-ci.

8. Système de support selon l'une des revendications 1 à 7, **caractérisé en ce que** la couche de support (30) et/ou la couche d'adhésif (32) du patch de support (18) présente une valeur de MVTR de plus de 400 g/m² de perméabilité à l'eau en 24 h.

9. Système de support selon l'une des revendications 1 à 8, **caractérisé en ce que** la liaison adhésive structurale (38) comporte un adhésif structural (40) à base d'adhésif cyanoacrylate, époxyde ou polyuréthane.

10. Système de support selon l'une des revendications 1 à 9, **caractérisé en ce que** l'adhésif sensible à la pression (34) et l'adhésif structural (40) présentent une composition biocompatible autorisée pour des applications médicales.

11. Système de support selon l'une des revendications 1 à 10, **caractérisé en ce que** la plate-forme de montage (20) est formée à partir d'une pièce moulée en matière plastique de préférence à base de polycarbonate.

12. Procédé pour la production d'un système de support pour un objet (12) porté par un corps selon l'une des revendications précédentes, comportant les étapes suivantes :
- préparation d'un patch de support (18), de préférence sous forme de pièce estampée, constitué d'une couche de support (30), d'une couche d'adhésif (32) et éventuellement d'une feuille détachable (22), dans lequel la couche d'adhésif (32) comprend une charge d'adhésif sensible à la pression allant de 80 à 120 g/m²,
- application d'un adhésif structural (40) sous forme de chenille d'adhésif sur une zone d'assemblage (36) d'une plate-forme de montage (20),
- mise en contact de la zone d'assemblage (36) avec la face supérieure, opposée à la peau, du patch de support (18) avec formation d'une liaison adhésive structurale (38), dans lequel la face supérieure de la couche de support (30) est reliée dans le même plan à une surface d'assemblage inférieure, lui faisant face, de la plate-forme de montage (20).

13. Procédé selon la revendication 12, **caractérisé en ce que** le patch de support (18) est pressé contre la plate-forme de montage (20) lors du durcissement de l'adhésif structural (40).

14. Procédé selon la revendication 12 ou la revendication 13, **caractérisé en ce que** l'adhésif structural (40) est durci en accéléré de préférence par rayonnement.

15. Système de support (10) selon l'une des revendications 1 à 11 destiné à être utilisé pour au moins une application parmi le groupe suivant
- surveillance sensorielle resp. détection d'un paramètre corporel,
- administration de substances actives, en particulier d'insuline sur ou à travers la peau (14),
- préparation d'un accès corporel ou d'une sortie corporelle,
- port d'un ornement corporel.
